# EUROPÄISCHE PATENTSCHRIFT

(11) **EP 0 489 716 B1**
(45) Veröffentlichungstag und Bekanntmachung des Hinweises auf die Patenterteilung: **09.11.1994**
(21) Anmeldenummer: 92103325.4
(22) Anmeldetag: 18.05.1988
(51) Int. Cl.: A61M 13/00, G05D 16/20

(54) **Gerät zum Insufflieren von Gas in eine Körperhöhle**
Device for insufflating gas in a body cavity
Appareil pour insuffler du gaz dans des cavités corporelles

(30) Priorität: 04.06.1987 DE 3718717
(43) Veröffentlichungstag der Anmeldung: 10.06.1992
(62) Teilanmeldung aus: 88107903.2
(73) Patentinhaber: Richard Wolf GmbH, 75438 Knittlingen (DE)
(72) Erfinder: Baier, Manfred, W-7134 Knittlingen (DE); Schäfer, Roland, W-7518 Bretten-Dürrenbüchig (DE)
(74) Vertreter: Wilcken, Thomas, Dipl.-Ing.

(56) Entgegenhaltungen:
- DE-A- 2 611 698
- DE-A- 3 413 631

## Beschreibung

Die Erfindung geht aus von einem Gerät zum Insufflieren von Gas in eine Körperhöhle gemäß dem Oberbegriff des Anspruchs 1.

Ein spezielles Problem beim Insufflieren von Gas in eine Körperhöhle, beispielsweise während eines operativen Eingriffs, besteht darin, mit einem Gasflow einen konstanten intraabdominalen Druck innerhalb physiologisch vertretbarer Grenzen zu erzeugen und diesen Druck genau zu messen. Ein Gerät hierfür ist z.B. in der DE-A-26 11 698 beschrieben. Bei den bei diesen Geräten verwendeten Meßwandlern besteht die Neigung, daß der jeweilige Ausgangswert des Wandlers bei Nichtbelastung des Wandlers durch Temperaturänderungen innerhalb des Insufflationsgerätes verfälscht wird.

Demgemäß besteht die Aufgabe der Erfindung darin, demjenigen Teil des Gerätes, welcher die Gasflußmenge zu dem das Gas in die Körperhöhle leitenden Instrument regelt, nämlich die Flowregelung, so zu verbessern, daß die Neigung von Meßwandlern, die zur Umwandlung eines Fluiddruckes in ein elektrisches Signal verwendet werden, ihren Ausgangswert bei Nichtbelastung durch Temperaturänderungen innerhalb des Gerätes infolge Wärmeentwicklung oder auch durch Alterung zu verändern, vermieden ist.

Diese Aufgabe wird bei einem Gerät der einleitend angeführten Art durch die Merkmale des Patentanspruchs 1 gelöst.

Nach dieser Lösung werden die Auswirkungen von Temperaturänderungen innerhalb des Insufflationsgerätes durch einen ständigen Nullpunktabgleich kompensiert. Dazu ist die erfindungsgemäß spezielle Kompensationsschaltung in der Flowregelung vorgesehen. Diese Maßnahme hat den Vorteil, daß der Gasfluß stets exakt und unabhängig von der thermischen Offsetkennlinie oder alterungsbedingten Veränderungen des Meßwandlers geregelt werden kann.

Eine funktionssichere, einfach aufgebaute und kostengünstig herstellbare Ausgestaltung der Erfindung besteht darin, daß das Zeitglied ein RC-Glied ist, dessen Kapazität als Speicher dient, und daß der genannte Kontakt in die Verbindung zwischen dem genannten weiteren Operationsverstärker und dem Speicher geschaltet ist.

Die Erfindung ist nachstehend anhand der anliegenden Zeichnungen beispielsweise näher erläutert. Es zeigen:
- Figur 1: eine schematische Schaltungsanordnung des erfindungs gemäßen Gerätes, wobei im oberen Teil der Flowregelkreis und im unteren Teil ein Druckregelkreis dargestellt ist,
- Figur 2: eine Kompensationsschaltung zum Nullpunktabgleich eines Druckmeßwandlers, wie sie in dem Flowregelkreis nach Figur 1 eingesetzt wird.

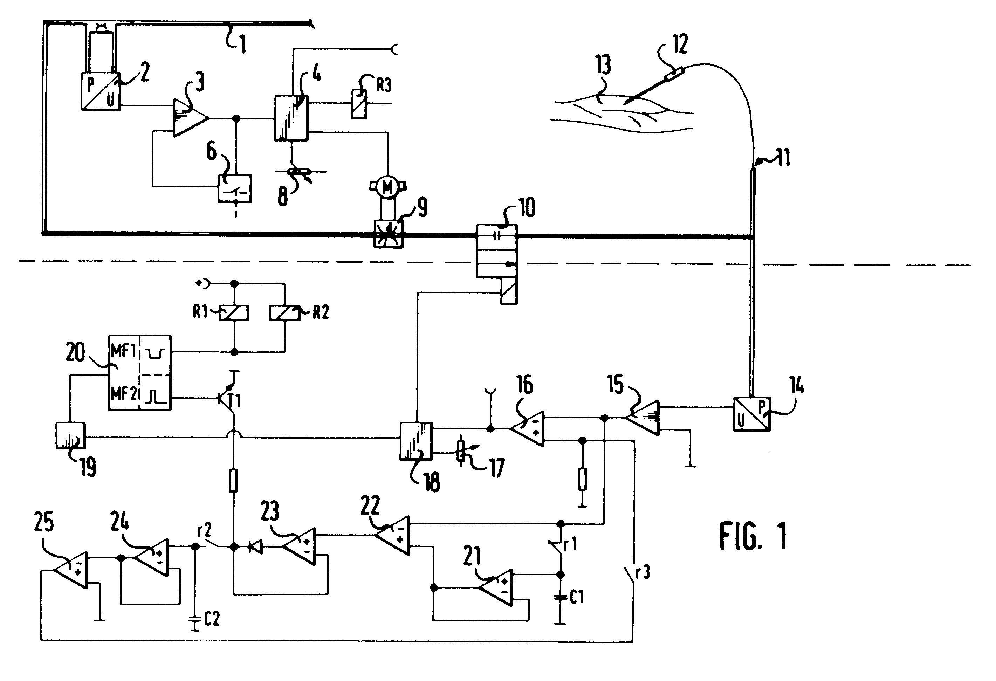

Gemäß Figur 1 wird Gas aus einem (nicht dargestellten) Druckbehälter über einen Gaseinlaß in die Gasleitung 1 des Geräts geleitet. Nach Durchgang durch die Flowregelung des Insufflators tritt das Gas aus einem Gasauslaß 11 über ein das Gas in eine Körperhöhle 13 leitendes Instrument 12, beispielsweise eine Veress-Nadel, in die Körperhöhle ein.

Die Flowregelung besteht aus einem eingangsseitig an die Gasleitung 1 angeschlossenen Differenzdruck-Meßwandler 2, dessen Ausgangssignal auf einen Eingang eines Operationsverstärkers (OP) 3 geführt ist. Dessen Ausgangssignal wird als Istwertsignal einer Regelelektronik 4 zugeführt, welche das Istwertsignal mit einem durch einen Einsteller 8 vorgewählten Sollwertsignal vergleicht. Bei einer Abweichung des Istwertes vom Sollwert des Gasflows trifft die Regelelektronik die notwendigen Maßnahmen, um den Gasflow mittels analogem Stellglied 9 an den Sollwert heranzuführen, das heißt, eine Drossel verengt oder erweitert den Durchlaß der Gasleitung 1. Über ein Ventil 10 gelangt der nunmehr kostante Gasflow an den Gasauslaß 11. Zwischen dem Ausgang des OP 3 und seinem zweiten Eingang ist eine Kompensationsschaltung 6 vorgesehen, die für einen Nullpunktabgleich des OP 3 bei nichtbelastetem Meßwandler 2 sorgt. Auf diese Kompensationsschaltung ist im einzelnen weiter unten eingegangen.

Der ausgangsseitig in der Gasleitung 1 herrschende Druck wird auf Seiten der Druckregelung von einem Druckmeßwandler 14 erfaßt, der den Druck in ein entsprechendes elektrisches Signal umwandelt. Ein dem Meßwandler 14 nachgeschalteter OP 15 führt das Meßsignal an den einen Eingang eines OP16, dessen Ausgang mit einer (nicht dargestellten) Druckanzeige sowie mit einer Auswerteelektronik 18 verbunden ist. Diese Auswerteelektronik 18 vergleicht das momentane Istwertsignal am Ausgang des OP16 mit einem Sollwertsignal, das mittels eines Einstellers 17 auszuwählen ist. Die Auswerteelektronik 18 ist mit dem Ventil 10 verbunden, welches in Abhängigkeit von dem Resultat des Vergleiches zwischen dem Soll- und Istwertsignal in der Elektronik 18 in der Weise gesteuert wird, daß der Gasflow in der Gasleitung 1 bei Erreichen des Solldruckes in der Körperhöhle 13, das heißt also bei Gleichheit zwischen dem Istwert- und dem Sollwertsignal in 18, abeschaltet wird.

Im Betrieb geht das Gerät zu verschiedenen Zeitpunkten vom Flow-Aus-Zustand in den Flow-Ein-Zustand über, etwa beim Einschalten, bei Konstanthalten des Solldrucks in der Körperhöhle 13, das durch kurze Insufflationsstöße geschieht, oder bei Veränderungen des Strömungswiderstandes des Drucksystems infolge eines Wechsels des Instrumentes 12 oder Flußschwankungen.

Eine an die Auswertelektronik 18 gekoppelte Steuerelektronik 19 erkennt und steuert diese Aus-Ein-Zustände. Ihr nachgeschaltet sind zwei Monoflops MF1 und MF2 im Bauteil 20. Beim Übergang vom Flow-Aus-Zustand des Gerätes in den Flow-Ein-Zustand wird in der Steuerelektronik eine ansteigende Impulsflanke erzeugt, die ihrerseits die nachgeschalteten Monoflops MF1 und MF2 triggert. Dem Monoflop MF1 nachgeschaltet sind zwei Relais R1 und R2, die für die Zeitdauer des Impulses in MF1 aktiviert werden. Die Relais R1 und R2 öffnen bzw. schließen zwei Schalter r1 bzw. r2 in einer Kompensationsschaltung, wie weiter unten beschrieben ist. Mit dem von MF2 erzeugten Impuls, dessen Impulsdauer wesentlich kürzer als die des Impulses im MF1 ist, wird ein nachgeschalteter Transistor T1 durchgeschaltet. Die Funktion des T1 ist weiter unten beschrieben.

Das Ausgangssignal des OP 15 wird neben der direkten Zuleitung an den OP 16 gleichzeitig der erwähnten Kompensationsschaltung zugeführt. Zum einen wird es direkt auf einen Eingang eines OP 22 geführt, zum anderen ist es über einen durch das Relais R1 gesteuerten Schalter r1 auf einen als Impedanzwandler geschalteten OP 21 mit hohem Eingangswiderstand und geringem Ausgangswiderstand sowie auch einen zwischen dem Eingang des OP 21 und Masse geschalteten Kondensator C1 schaltbar. Der Ausgang des OP 21 ist an dem zweiten Eingang des OP 22 angeschlossen. OP22 verstärkt die Spannungsdifferenz, deren Spitzenwert über den nachgeschalteten OP23 mit Diode an dessen Ausgang erhalten wird. Der Ausgang des OP 23 mit Diode ist zum einen an den erwähnten Transistor T1 angeschlossen, zum anderen ist er über einen durch das Relais R2 gesteuerten Schalter r2 auf einen als Impedanzwandler geschalteten OP24 sowie auf einen zwischen dem Eingang des OP 24 und Masse geschalteten Kondensator C2 schaltbar. Der Ausgang des OP 24 wird im nachgeschalteten OP 25 verstärkt und über einen Schalter r3, der im Flow-Aus-Zustand des Gerätes geöffnet und im Flow-Ein-Zustand geschlossen ist, an den anderen Eingang des OP 16 geführt.

Im einzelnen funktioniert die Druckmessung mit der beschriebenen Schaltungsanordnung wie folgt.

Ist der Gasflow abgeschaltet, so steht in der Gasleitung 1 ausgangsseitig der statische intraabdominale Druck, der keinerlei Verfälschungen durch einen Fließdruck erfährt. Dieser Wert wird in Form einer entsprechenden Spannung über OP 15 und OP 16 auf die Druckanzeige und gleichzeitig auf die Kompensationsschaltung gebracht, wobei der Wert über den in diesem Betriebszustand geschlossenen Schalter r1 auf den Kondensator C1 geführt und dort gespeichert wird. Der Wert wird über OP 21 an den Eingang des OP 22 geführt, dessen zweiter Eingang direkt mit der Meßspannung verbunden ist. Da eingangsseitig am OP 22 beide Werte gleich sind, erfolgt in dieser Phase keine weitere Signalverarbeitung.

Wenn nun der Gasflow wieder zugeschaltet wird, etwa wenn der Istwert des statischen intraabdominalen Druckes unter dem Sollwert liegt, werden die Schalter r1 und 2 in dieser Zuschaltphase durch die von dem Monoflop MF1 gesteuerten Relais R1 undR2 kurz geöffnet bzw. geschlossen. Gleichzeitig wird durch den vom Monoflop MF2 erzeugten Impuls über T1 und den nun geschlossenen Schalter r2 der Kondensator C2 kurzgeschlossen, damit ein in C2 bei einem vorangegangenen Zuschalten des Gasflows gespeicherter Spannungswert gelöscht wird. Mit dem Zuschalten wird der Schalter r3 geschlossen. In dieser Phase steht an dem Meßwandler 14 der statische intraabdominale Druck sowie der dynamische Druck des Gasflows, dessen Wert es zu kompensieren gilt. Der aufgrund des erhöhten Druckwertes erhöhte Spannungswert an OP 16 wird an OP 22 durchgeführt. Da in dieser Phase der Schalter r1 geöffnet ist, wird die zuvor an C1 liegende Spannung, dem bei Flow-Aus-Zustand anlag und den Wert des statischen intraabdominalen Druckes repräsentiert, über OP 21 weiterhin an den zweiten Eingang von OP 22 geführt. Dementsprechend repräsentiert die Spannung am Ausgang vom OP 22 nur den dynamischen Teil des am Wandler anstehenden Gesamtdruckes, also den Insufflationsdruck. Der Spitzenwert der Ausgangsspannung vom OP 22 wird über OP 23 und Diode erhalten. Mit diesem Spitzenwert wird der Kondensator C2 über den in dieser Phase geschlossenen Schalter r2 geladen. Er wird über OP 24 und OP 25 und geschlossenem Schalter r3 als Offset an den zweiten Eingang vom OP16 geführt, an dessen Ausgang nunmehr lediglich der den statischen Anteil des Gesamtdruckes repräsentierende Spannungswert erscheint und zur Anzeige gebracht wird.

Die Dauer dieser Übergangsphase liegt im Millisekundenbereich. Danach sind die Relais R1 und R2 wieder inaktiv, das heißt, Schalter r1 wieder geschlossen und Schalter r2 wieder geöffnet. Der am Kondensator C2 gespeicherte Spannungswert wird weiterhin bis zum Ende der Insufflationsphase als Offset an OP 16 geführt. Der Wert entspricht genau der Überhöhung, die das angeschlossene Instrument 12 verursacht. Meßwerte, die nun betraglich höher als dieser Wert liegen, können nur durch einen externen Druck verursacht sein. Hier ist dies der intraabdominale Druck in der Körperhöhle 13, also der Druck, der für die Regelung des Gerätes auf den eingestellten Solldruck maßgebend ist.

Eine vorteilhafte Weiterbildung der Flowregelung besteht, wie oben erwähnt, in der Zwischenschaltung einer Kompensationsschaltung 6. Diese Kompensationsschaltung dient zum Nullabgleich des Meßwandlers 2 in unbelastetem Zustand.

Wenn der Meßwandler 2 unbelastet ist, sollte sich an seinem Ausgang im Normalfall eine Spannung von 0 Volt einstellen. Dies kann aber aufgrund von Temperaturänderungen innehalb des Gerätes infolge Wärmeentwicklung oder auch alterungsbedingt anders sein.

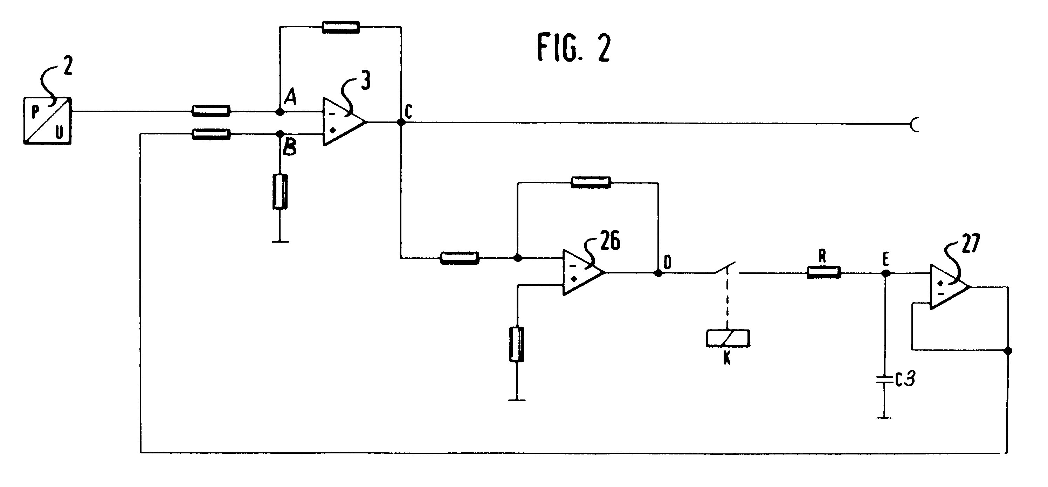

In der Schaltung nach Figur 2 wird eine von dem Druckmeßwandler 2 erzeugte Spannung an einen Eingang eines OP 3 geführt. Der Ausgang von OP 3 wird auf eine Verstärkerschaltung mit OP 26 geführt, die eine hohe Verstärkung aufweist. Das Ausgangssignal von OP 26 ist über einen Kontakt K, der immer dann geschlossen wird, wenn der Meßwandler 2 unbelastet ist, über ein Zeitglied RC auf einen elektrischen Speicher (in Figur 2: C3) schaltbar. Im weiteren Verlauf wird das Signal über einen OP 27, der als Impedanzwandler geschaltet ist, an den zweiten Eingang des OP 3 zurückgeführt.

Die Kompensation wird mit dem Schließen des Kontaktes K aktiviert, also immer, wenn der Meßwandler 2 unbelastet ist. Dies ist dann der Fall, wenn der eingestellte Sollwert des intraabdominalen Druckes erreicht ist und der Gasflow abgeschaltet wird, da es in diesem Fall auch keinen Druckabfall mehr gibt, und gleich beim Einschlaten des Gerätes.

Die Funktionsweise der Schaltung ist anhand des folgenden Beispieles verdeutlicht:
Der Meßwandler 2 sei unbelastet und der Kontakt K demnach geschlossen. Am Ausgang des Meßwandlers 2 steht eine Spannung von +1 Volt an, am Punkt B eine Spannung von +0,2 Volt. Der Verstärkungsfaktor von OP 3 betrage 1, derjenige von OP 26 betrage 1000.

Unter diesen Voraussetzungen ist Punkt A also um 0,8 Volt positiver als Punkt B. Demnach liegt Punkt C am Ausgang des OP 3 betragsmäßig ebenfalls auf 0,8 Volt, schaltungsbedingt aber mit negativen Vorzeichen, also minus 0,8 Volt. Da OP 26 eine hohe Verstärkung hat, würde dieser in die Sättigung fahren auf etwa 11 Volt mit positivem Vorzeichen wegen des invertierenden Verhaltens von OP 26. Mit dem am Punkt D stehenden Wert wird der Kondensator C3 über den Widerstand R geladen, wobei das RC-Glied einen langsameren Anstieg der Spannung an den Punkten E und B als am Punkt D bewirkt. Dieselbe Spannung wird über OP 27 ohne Vorzeichenumkehr zum Punkt B geführt. Wenn die Spannung an den Punkten D, E und B den zu kompensierenden Wert von +1 Volt am Punkt A erreicht hat, hört OP 26 auf, in die Sättigung von +11 Volt zu fahren.

Da dann die Spannungswerte an den Punkten A und B gleich sind, liegt Punkt C auf Null Volt. Da OP 26 nun keine Spannung zu verstärken hat, liegt Punkt D ebenfalls auf Null Volt. An dem Kondensator C3 liegt nun eine Spannung von +1 Volt, so daß er sich über den OP 26 wieder entladen könnte. Dies wird aber durch die geschlossene Regelschleife, wie beschrieben, ausgeglichen.

Wenn sich nun der Kontakt K öffnet, steht an dem Kondensator C3 weiterhin die Spannung von +1 Volt an, die benötigt wird, um den Punkt C auf Null Volt auszuregeln. Tritt nun an dem Punkt A infolge eines Druckabfalls am Meßwandler 2 eine Spannung abweichend von +1 Volt auf, wird diese als Meßspannung angesehen und weiter verarbeitet.

Der offene Regelkreis unterbindet eine weitere Kompensation. Die zuvor erhaltene Kompensationsspannung am Kondensator C3 bleibt je nach Dimensionierung von R, C3 und OP 27 für 5 bis 60 Minuten am Punkt B erhalten. Der beschriebene Ausregelvorgang läuft im Bereich von Milisekunden ab.

Die Schaltung kann auch dafür eingesetzt werden, um einen virtuellen Nullpunkt zu erzeugen. Beispielsweise kann ein bestimmter Druck auf den Meßwandler 2 gegeben und der daraus resultierende Spannungswert am Punkt A durch kurzes Schließen des Kontaktes K ausgeglichen und als Nullpunkt angesehen werden. Erst von diesem bestimmten Druck abweichende Drücke wären dann auszuwerten.

## Patentansprüche

1. Gerät zum Insufflieren von Gas in eine Korperhöhle mit einem Meßwandler (2), zwei Operations verstärkern (3, 26), einem Regelkreis (4), einer Gasleitung (1) mit einem Stellglied (9) und einem Kontakt (K), bei dem der Flow des zur Körperhöhle geleiteten Gases mit dem auf eine Nullstellung kompensierbaren Meßwandler (2) erfaßt und mit dem sich ergebenden Ausgangssignal des Meßwandlers der Regelkreis (4) angesteuert wird, um den Gasflow mit dem in der Gasleitung (1) liegenden Stellglied (9) auf einen Sollwert einzuregeln, dadurch gekennzeichnet daß es einen elektrischen Speicher (C3) aufweist, daß ein bei eingangsseitig unbelastetem Meßwandler (2) auftretendes Leerlaufsignal dem einen Eingang des einen Operationsverstärkers (3) zugeführt wird, der aus den Beträgen dieses Leerlaufsignals und eines seinem anderen Eingang zugeführten Kompensationssignals ein Differenzsignal bildet, daß das Ausgangssignal des genannten einen Operationsverstärkers (3) mit dem weiteren Operationsverstärker (26) verstärkt und mit dessen Ausgangssignal über ein Zeitglied (RC) der elektrische Speicher (C3) aufgeladen wird, der mit dem genannten anderen Eingang des einen Operationsverstärkers (3) bleibend in Verbindung steht, und daß der Speicher vom Ausgang dieses Operationsverstärkers durch Öffnen des Kontaktes (K) abtrennbar ist.

2. Gerät nach Anspruch 1, dadurch gekennzeichnet, daß das Zeitglied ein RC-Glied ist, dessen Kapazität (C3) als Speicher dient.

3. Gerät nach Anspruch 1 oder 2, dadurch gekennzeichnet, daß der Kontakt (K) in die Verbindung zwischen dem genannten weiteren Operationsverstärker (26) und dem Speicher (C3) geschaltet ist.

## Claims

1. An instrument for the insufflation of gas into a body cavity, with a measuring transducer (2), two operational amplifiers (3,26), a control circuit (4), a gas line (1) with a correcting element (9) and a contact (K), in which the flow of the gas which is conducted to the body cavity is picked up by the measuring transducer (2), which is able to be compensated to a zero setting, and with the resulting output signal of the measuring transducer the control circuit (4) is controlled, in order to regulate the gas flow to a nominal value with the correcting element (9) lying in the gas line (1), characterised in that it has an electric accumulator (C3), that an idling signal, occurring when the measuring transducer (2) is unloaded on the input side, is supplied to one input of one operational amplifier (3), which forms a differential signal from the amounts of this idling signal and of a compensation signal supplied to its other input, that the output signal of the said one operational amplifier (3) is amplified by the further operational amplifier (26) and with its output signal, via a timing element (RC), the electric accumulator (C3) is loaded, which is permanently connected with said other input of the one operational amplifier (3), and that the accumulator is able to be separated from the output of this operational amplifier by opening the contact (K).

2. An instrument according to Claim 1, characterised in that the timing element is a RC-element, the capacitor (C3) of which serves as accumulator.

3. An instrument according to Claim 1 or 2, characterised in that the contact (K) is connected into the connection between the said further operational amplifier (26) and the accumulator (C3).

## Revendications

1. Appareil pour insuffler du gaz dans une cavité corporelle, comprenant un transducteur (2), deux amplificateurs opérationnels (3, 26), un circuit de réglage (4), un conduit (1) pour le gaz équipé d'un composant de réglage (9) et d'un contact (K), dans lequel l'écoulement du gaz guidé en direction de la cavité corporelle est enregistré par le transducteur (2) qui peut être compensé par un mesurage par zéro, le circuit de réglage (4) étant amorcé avec le signal de sortie fourni par le transducteur pour régler l'écoulement de gaz à une valeur de consigne avec le composant de réglage (9) disposé dans le conduit (1) pour le gaz, caractérisé en ce qu'il présente une mémoire électrique (C3), en ce qu'un signal de marche à vide, qui apparaît lorsque le transducteur n'est pas sollicité côté entrée, est acheminé à une première entrée du premier amplificateur opérationnel (3), qui forme un signal de différence à partir des valeurs de ce signal de marche à vide et d'un signal de compensation acheminé à son autre entrée, en ce que le signal de sortie du premier amplificateur opérationnel mentionné (3) est amplifié avec celui de l'autre amplificateur opérationnel (26), la mémoire électrique (C3) étant chargée avec le signal de sortie de ce dernier via un relais de temporisation (RC), la mémoire se trouvant en liaison constante avec l'autre entrée mentionnée du premier amplificateur opérationnel (3), et en ce que la mémoire peut être séparée de la sortie de cet amplificateur opérationnel par l'ouverture du contact (K).

2. Appareil selon la revendication 1, caractérisé en ce que le relais de temporisation est un composant RC dont la capacité (C3) sert de mémoire.

3. Appareil selon la revendication 1 ou 2, caractérisé en ce que le contact (K) est monté dans la liaison entre le deuxième amplificateur opérationnel mentionné (26) et la mémoire (C3).
